# EUROPEAN PATENT SPECIFICATION

(11) **EP 1 154 723 B1**
(45) Date of publication and mention of the grant of the patent: **19.12.2007**
(21) Application number: 00904626.9
(22) Date of filing: 28.01.2000
(51) Int. Cl.: A61B 17/00, A61B 17/12, A61L 24/04, A61L 31/04

(54) **CYANOACRYLATES COMPRISING INHIBITORS AND AN OPACIFYING AGENT**
CYANOACRYLATE UMFASSEND INHIBITOREN UND EIN TRÜBUNGSMITTEL
CYANOACRYLATES COMPRENANT DES INHIBITEURS ET UN AGENT D'OPACIFICATION

(30) Priority: 29.01.1999 US 241368
(43) Date of publication of application: 21.11.2001
(73) Proprietor: BOSTON SCIENTIFIC CORPORATION, Natick, Massachusetts 01760 (US)
(72) Inventor: KRALL, Robert, E., Alpine, CA 91901 (US); KERBER, Charles, W., La Mesa, CA 91941 (US); KNOX, Kimberly, La Mesa, CA 91941 (US)
(74) Representative: Vossius & Partner
(86) International application number: PCT/US2000/002262
(87) International publication number: WO 2000/044287

(56) References cited:
- WO-A-97/07783
- WO-A-99/42535
- US-A- 4 313 865
- US-A- 5 695 480
- US-A- 5 702 361
- US-A- 5 759 194

## Description

### FIELD OF THE INVENTION

This invention relates to cyanoacrylate compositions useful as medical devices.

### BACKGROUND OF THE INVENTION

Cyanoacrylate tissue adhesives have been in clinical endovascular use since the 1970's. Liquid acrylics are extremely useful as endovascular embolic agents because of their ability to create permanent vascular occlusion. They may, however, be difficult to use technically as they have a variable and sometime unpredictable polymerization time based on the operator selection of an acrylic mix with either iodinated oil or glacial acetic acid. The appropriate choice of polymerization time depends on a number of variables, including the transit time between arterial and venous elements in the embolic target, the target volume, the architecture of the target, for example, a fistula versus nidus, which affects the relative endovascular turbulence, and the method of injection (bolus, full column, or wedge-flow arrest). Typical complications associated with the use of liquid acrylics for embolization occur when there is occlusion of normal arterial branches or acrylic penetration into critical venous outflow channels. Additionally, reflux of acrylic around the delivery catheter tip can result in permanent endovascular catheter adhesion, which may require permanent catheter implantation. Overzealous attempts at withdrawal can produce catheter fracture (and resultant embolization of flow-directable distal catheter segment), vascular damage with resultant dissection/occlusion, or avulsion of the involved vascular pedicle (with resultant subarachnoid hemorrhage).

Alkyl alpha cyanoacrylates are a homologous series of organic molecules which polymerize and can adhere to moist living tissues. The methyl homolog has been used in homeostasis and non-suture closure since 1960, but its histoxicity severely limited its clinical usefulness. The synthesis of longer alkyl chain homologs and the evaluation of these in various animal species have shown that the histoxicity of cyanoacrylates could be diminished without sacrificing their hemostatic and tissue bonding properties. Extensive animal studies have been completed using n-butyl and isobutyl homologs, and preliminary human trials have been undertaken.

Polymerization speed is another function of chain length. It has been reported that homologs with six or more carbon atoms on the alkyl chain polymerize almost immediately upon contact with moist tissues. The *n*-butyl and isobutyl monomers require from four to 15 seconds, while the methyl homolog remains as a monomer for 30 to 55 seconds. The ability to wet and spread easily over the surface of an anticoagulated blood film is common to homologs with alkyl chains containing four or more carbon atoms. The ethyl and propyl derivatives wet and spread poorly, and the methyl not at all.

Since the advent of NBCA (n-butyl-2-cyanoacrylate), there has been very little advancement in the science of "superglue" embolization of vascular structures, primarily arteriovenous malformations (AVMs). Certain properties of superglue are advantageous for embolization, such as adhesion, the ability transform from a liquid or solid state and rapid polymerization. However, these properties can be detrimental when present to an excessive degree, in particular, adhesion which can result in permanent catheter fixation. Rapid polymerization allows the material to set in flowing blood without passing through small channels into venous structures. However, rapid polymerization may also release amounts of heat that can cause damage to the surrounding tissue, for example, brain tissue.

Hydrophilic catheter coatings have been developed in the hope of reducing the risk of inadvertent endovascular catheter fixation during embolization due to reduced bond strength between the hydrophillically coated catheter and the adhesive. However, micro catheter cyanoacrylate adhesion remains a problem during intravascular embolization. Inadvertent gluing of the catheter tip onto the artery is a well recognized and distressing complication. Vessel rupture or occlusive embolization of a detached catheter tip may occur if excessive force is used to attempt to retrieve the catheter. Fortunately, permanent intra vascular catheter fixation is usually well tolerated, nonetheless this remains a highly undesirable event. An *in vitro* study has shown that recently available hydrophilic micro catheter coatings decrease catheter adhesion of both pure normal butyl cyanoacrylate and mixtures of normal butyl cyanoacrylate and ethiodized oil. Although hydrophilically coated catheters have the potential of decreasing the occurrence of inadvertent endovascular catheter fixation, the level of operator proficiency and experience, and perhaps most importantly, the actual adhesive composition that is used stills play a major role in these events.

There exists a continuing unmet need for a composition that has the correct amount of cohesiveness, produces a robust rubbery casting, is tolerated by the body, can trigger the appropriate amount of tissue inflammation response and is radiopaque.

It has now been surprisingly found that such a composition exists that has the requisite combination of properties in cohesion, stability, body tolerance, low catheter adhesion and radiopacity.

### SUMMARY OF THE INVENTION

A composition comprising a combination of a monomer component comprised of an alkyl cyanoacrylate and at least one inhibitor; and a second component comprised of an alkyl cyanoacrylate monomer, an alkyl esterified fatty acid and an opacifying agent where said composition polymerizes when said composition contacts a non-ionic environment. The composition is useful for filling an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition; the composition is also useful for adhering tissue to tissue, or adhering tissue to a device. The composition has the property of polymerizing when it comes in contact with a non-ionic environment, or when it is deployed *in situ* in an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition.

### BRIEF DESCRIPTION OF THE DRAWINGS

No drawing are included.

### DETAILED DESCRIPTION OF THE INVENTION

The present invention provides a composition comprising a combination of a monomer component comprised of an alkyl cyanoacrylate, at least one inhibitor and a second component that functions as an opacifying agent and polymerization retardant. The composition is useful for filling, occluding, partially filling or partially occluding an unfilled volume or space in a mass ("a space"). In particular, the composition is useful for filling an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition; the composition is also useful for adhering tissue to tissue, or adhering tissue to a device. The composition has the property of polymerizing when it comes in contact with a non-ionic environment, or when it is deployed *in situ* in an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition.

Another aspect of the present embodiment is where the second component is comprised of a halogenated oil. Preferred are iodinated and brominated oils, such as Ethiodol^{®}, Lipiodol and Pantopaque. Most preferred is Ethiodol^{®}

One embodiment of the present invention is where the second component is Ethiodol^{®}.

Another aspect of the present embodiment is where the second component is comprised of an alkyl cyanoacrylate monomer, an alkyl esterified fatty acid and an opacifying agent.

Another aspect of the present embodiment is where the monomer component is comprised of an alkyl cyanoacrylate monomer, and at least two inhibitors, a preferred aspect is where the monomer component is comprised of an alkyl cyanoacrylate and at least, three inhibitors, an especially preferred aspect is where the monomer component is comprised of 2-hexyl cyanoacrylate and at least one inhibitor. An especially preferred aspect is where the monomer component is comprised of 2-hexyl cyanoacrylate and at least two inhibitors. A most especially preferred aspect is where the monomer component is comprised of 2-hexyl cyanoacrylate, and three inhibitors, particularly, most especially preferred is the aspect where one of the inhibitors is selected from hydroquinone, *p*-methoxyphenol or phosphoric acid. A most especially preferred aspect is where the monomer component is comprised of 2-hexyl cyanoacrylate, and three inhibitors, where the three inhibitors are hydroquinone, *p*-methoxyphenol and phosphoric acid. The quantity of inhibitors used is measured in terms of parts per million of alkyl cyanoacrylate. Preferably, hydroquinone is in the range of about 50 to 150 parts per million (PPM), *p*-methoxyphenol in the range of about 50 to 150 PPM, and phosphoric acid in the range of about 125 to 375 PPM, more preferred is hydroquinone in the range of about 75 to 125 PPM, *p*-methoxyphenol in the range of about 75 to 125 PPM, and phosphoric acid in the range of about 187.5 to 312.5 PPM, and most preferred is hydroquinone in the range of about 95 to 105 PPM, *p*-methoxyphenol in the range of about 95 to 105 PPM, and phosphoric acid in the range of about 200 to 300 PPM.

An especially preferred embodiment of the present invention is a composition comprised of the present monomer component, and a second component comprising 2-hexyl cyanoacrylate monomer, an alkyl esterified fatty acid and an opacifying agent, most especially preferred is where the alkyl esterified fatty acid is ethyl myristate, also most especially preferred is where the opacifying agent is gold.

Another embodiment of the present invention describes a method for filling, occluding, partially filling or partially occluding an unfilled volume or space in a mass. The types of unfilled volumes or spaces within the scope of the present invention includes, but are not limited to the following instances.

For example, one aspect of the present embodiment is a method of filling, occluding, partially filling or partially occluding an existing space, such as, a lumen of a passageway in the body, e.g., a blood vessel, a duct, an aneurysm, or a fistula. Examples of the types treatments covered by this method of use, include but are not limited to the following. The present invention is useful as a method of treating arteriovenous malformations (AVM) where the blood vessel(s) that feed the AVM are occluded thereby cutting off the blood supply to the AVM. The present invention is useful as a method to ablate diseased or undesired tissue by cutting off the tissue's blood supply. In particular, the present invention is useful as a method of treating a tumor having a discrete blood supply, where the blood vessel(s) that feed the tumor are occluded thereby cutting off the blood supply to the tumor resulting in diminished growth or death of the tumor. The present invention is useful as a method of preventing or mitigating the development of an aneurysm by creating a partial occlusion at a location in the blood vessel selected to modify the fluid dynamics within the vessel to mitigate the formation or development of an aneurysm. The present invention is useful as a non-surgical method of treating symptomatic uterine leiomyomas by embolizing/occluding the uterine artery. This method has been reported using a non alkyl cyanoacrylate composition in J.Vascular and Intervention Radiology, 10:891-894, July-August 1999. The present invention is useful as a method of sterilizing a female mammal by occluding the fallopian tubes thereby preventing the passage of the eggs from the ovaries to the uterus. The use of an occluding agent to sterilize a female mammal is disclosed in U.S. Patent No. 5,989,580 "Method of Sterilizing Female Mammals," herein incorporated by reference. The methods disclosed in this patent can be advantageously applied using the compositions of the present invention, and are within the scope of the present invention. The present invention is useful for obliterating the left atrial appendage. The left atrial appendage is derived from the left wall of the primary atrium. It has been observed that patients with atrial fibrillation have a predilection for thrombus to form in the in the left atrial appendage. A review of this condition and the current status of treatment is disclosed in the article, "Left Atrial Appendage: structure, function, and role in thromboembolism" N.M. Al-Saady, et. al. The present invention provides an advantageous method of obliterating the left atrial appendage.

Another aspect of the present embodiment is a method of filling, occluding, partially filling or partially occluding a space created by an external device, such as, a catheter balloon. Examples of the types of treatments covered by this method of use include, but are not limited to the following. The present invention is useful as a method of treating an aneurysm by filling the space within the aneurysm with a composition of the present invention, where the composition polymerizes in the space within the aneurysm, thereby preventing the rupture of the aneurysm. This treatment can be effected using the present invention with any number of catheters, catheter coils, catheter wires or catheter balloons commercially available. Examples of such devices are commercially available from sources. For instance, Micro Therapeutics, Inc., 2 Goodyear, Irvine, California 92618, markets a line of medical devices, such as, the Rebar™ Micro Catheter, Equinox™ Occlusion Balloon System and SilverSpeed™ guidewires. Similarly, U.S. Patent No. 5,882,334 "Balloon/delivery Catheter Assembly with Adjustable Balloon Positioning," assigned to Target Therapeutics, Inc., and incorporated herein by reference, is directed to a catheter assembly for delivering compositions, such as, those of the present invention.

Another aspect of the present embodiment is a method of filling, occluding, partially filling or partially occluding a space created or resulting from a procedure, such as with the excision of tissue, or insufflation. Examples of the types of treatments covered by this method of use include, but are not limited to the following. The present invention is useful as a method of treating or mitigating capillary oozing.

Another aspect of the present embodiment is a method of filling, occluding, partially filling or partially occluding a space created by the placement or implantation of an object, such as, a medical device. Examples of the types of uses covered by this method of use include, but are not limited to the following. The present invention is useful as a method of restoring the normal fluid dynamics at the peripheral edges of a vascular stent by filling the dead spaces between the stent and the lumen wall created by the implantation of the stent.

Another aspect of the present embodiment is a method of filling, occluding, partially filling or partially occluding a space created by the composition itself, such as, where the composition is used as a bulking agent. Examples of the types of uses covered by this method of use include, but are not limited to the following. For example, a method of recreating the normal contours to skin following an adverse event, such as, physical trauma.

Another embodiment of the present invention provides a method of affixing therapeutics, chemotherapeutics, radiation delivery devices, gene therapy compositions to a desired location where the active agents can be advantageously maintained in proximity to the desired location. The active agent is then release gradually as the resultant aggregate structure from the composition of the present invention is biodegraded. Alternatively, the composition of the present invention can be modified to allow for a specific rate of delivery. This use is particularly beneficial in the treatment of tumors that are ideally treated by localized dosages of chemotherapy or radiation. An advantage of this method is that the patient would not be subjected to as large of a dose of the therapeutic or radiation as would be necessary, if the therapeutic or radiation was administered on a systemic basis. Another advantageous use the present invention is for the delivery of DNA compositions used in gene therapy. A long standing problem in the gene therapy arts has been the inability of practitioners to deliver the DNA therapeutic to the locales in the body most ideally suited for the treatment. The present invention provides a method of affixing the DNA composition at a desired site, where the active agent is then slowly released over a period time as the composition of the present invention biodegrades. Alternatively, a composition of the present invention can be modified to release the active agent in a controlled delivery manner.

Another embodiment of the present invention provides a method of utilizing magnetically controlled particles inbedded in a composition of the present invention to deploy the composition to a desired location, "Magnetic Probe for the Stereotaxic Thrombosis of Intracranial Aneurysms," Alksne, J.F., et. al, Journal of Neurology, Neurosurgery and Psychiatry, 1967 April, 30(2):159-62; "Magnetically Controlled Focal Intravascular Thrombosis in Dogs" Alksne, J.F., et. al, Journal of Neurosurgery, 1966 Nov, 25(5):516-25; "Thrombosis of Intracranial Aneurysms - An experimental approach utilizing magnetically controlled iron particles" Alksne, J.F., et. al, Radiology 1966 Feb. 86(2):342-3

Another embodiment of the present invention provides a method of adhering, joining, connecting or affixing a first section of tissue to a second section of tissue. Examples of the types of uses covered by this method of use include, but are not limited to the following. The present invention is useful as a method of adhering, joining, or connecting two blood vessels, e.g., anastimosis, where blood vessels are quickly and efficiently adhered, joined or connected, under surgical conditions without the use of sutures or staples. The present invention is useful as a method of treating primary wounds or wounds that require immediate intervention, such as, trauma wounds, where the compositions of the present invention are used to temporarily close the wound to minimize the lost of fluids due to evaporation, and to mitigate infection.

Another embodiment of the present invention provides a method of adhering, joining, connecting, or affixing tissue to a non-tissue surface, such as a medical device. Examples of the types of uses covered by this method of use include, but are not limited to the following. The present invention is useful as a method of implanting or securing venous valves, replacement heart valves, or stents at their desired location.

The aforementioned uses are possible because the compositions of the present invention remain in a controllable state for a period of time in excess of 1 second after being deployed from an administration device. This property allows the practitioner to incrementally maneuver the deployment of the composition to its most ideal location, even when the composition has been partially deployed distal the the deployment device.

For instance, the compositions of the present invention have adequate cohesion to maintain its continuity once it is outside of the deployment device. Without adequate cohesion the composition would break into smaller aggregates dispersing into the blood flow.

For instance, the compositions of the present invention have appropriate adhesion properties so that when desired a deployed composition adheres to the immediate location where it is deployed so that the monomer is placed where it is desired.

The compositions of the present invention have polymerization rate, such that, the practitioner can effect the desired amount of penetration of the composition into a particular type of space. A composition that polymerizes too quickly would hinder penetration, conversely a composition that polymerizes too slowly would make it difficult to precisely place the polymerized composition resultant aggregate of the monomer.

Another embodiment of the present invention describes a method for selectively creating an embolic blockage in the lumen of a blood vessel, duct, fistula or other like body passageways.

Another embodiment of the present invention provides a method of treating arteriovenous malformation (AVM)

### DEFINITIONS

As used herein the terms "adhesion" or "adhesive" means the characteristic or tendency of a material to be attracted to the surface of a second material. Adhesion occurs as the result of interactions between two materials. Depending on the characteristics of the second material relative to the first material, adhesion may or may not occur. For a single material, e.g., the composition of the present invention, the presence of adhesion is demonstrated by a material sticking to the wall of a lumen of blood vessel, i.e., there is adhesion between the material and the lumen wall. Conversely, the absence of adhesion is demonstrated for the same material where a micro-catheter tip used to deposit the material can be removed from the material, i.e., there is little adhesion between the material and micro-catheter tip.

As used herein the term "alkyl" refers to a carbon chain of one to sixteen carbon atoms, where the carbon atoms can be linear or branched.

As used herein the term "non-ionic environment" refers to an environment that is devoid of charged ions, or where the charged ions are complexed with other molecules which effectively neutralize their charge. For example, a solution of water and a sugar, such as, dextrose, and blood, is an non-ionic environment.

As used herein the term "lower-alkyl" refers to a carbon chain of one to eight carbon atoms, where the carbon atoms can be linear or branched. Examples of lower-alkyl moieties include but are not limited to methyl, ethyl, *n*-butyl, isobutyl, pentyl, *n*-hexyl, 2-hexyl, *n*-heptyl, 2-heptyl, *n*-octyl and 2-octyl.

As used herein the term "branched alkyl" refers to a carbon chain of one to sixteen carbon atoms where the carbon chain contains at least one secondary or tertiary substituted carbon atom.

As used herein the term "branched lower-alkyl" refers to a carbon chain of one to eight carbon atoms where the carbon chain contains at least one secondary or tertiary substituted carbon atom, for example, 2-hexyl, isobutyl, 2-heptyl and 2-octyl.

As used herein the term "cohesion" or "cohesive" means the characteristic or tendency of a material to stick together to itself. For example, this characteristic is demonstrated by a material or composition remaining intact as a single mass when introduced into a stationary fluid, or a fluid stream in motion, such as, blood. Lack of cohesive integrity results in the composition breaking up into multiple smaller subunits.

As used herein the term "embolic agent" refers to a non-naturally occurring composition introduced into a body cavity or the lumen of a blood vessel, duct, fistula or other like body passageways for the purpose of forming an embolic block.

As used herein the term "embolic block" or "embolic blockage" or occlusion refers to the end result from the administration of a composition useful as an embolic agent. The resulting embolic block mechanically blocks, totally or partially, the lumen of a blood vessel, duct, fistula or other like body passageways; or in a like manner forms an occlusion within a cavity, such as an aneurysm.

As used herein the term "alkyl cyanoacrylate monomer" refers to the chemical entity of the general structure B₂C=C(CN)-C(O)O-R, where R is an alkyl moiety of one to sixteen carbon atoms, linear or branched, saturated or unsaturated, having the physical characteristic of being able to form the corresponding alkyl cyanoacrylate.

As used herein the term "alkyl cyanoacrylate polymer" means an oligomer or polymer resulting from the polymerization of a alkyl cyanoacrylate monomer.

As used herein the term "alkyl esterified fatty acid" means a fatty acid derivatized to form an ester functional group with a alkyl moiety, such as ethyl myristate. These compounds are formed with an alkyl moiety, such as, methyl, ethyl, propyl, butyl, pentyl, hexyl, heptyl, and octyl; and carboxylic acids with alkyl side chains ranging from 1 carbon, i.e., acetic acid, through to and including 17 carbons atoms in length, such as, proprionic, butyric, isobutyric, valeric, isovaleric, pivalic, lauric, myristic, palmitic and stearic acids.

As used herein the term opacifying agent" is compound or composition which selectively absorbs or deflects radiation making the material visible under x-ray, or any like imaging technique. Typically such agents include, iodinated oils, and brominated oils, as well as commercially available compositions, such as Pantopaque, Lipiodol and Ethiodol^{®}. These commercially available compositions acts as opacifying agents, and also dilute the amount of liquid monomer thereby slowing the rate of polymerization. In addition certain metals, such as, gold, platinum, tantalum, titanium, tungsten and barium sulfate and the like, have properties enabling them to act as opacifying agents.

As used herein the term "polymerization" refers to the chemical process where identical monomer units react chemically to form larger aggregates of said monomeric units as oligomers or polymers.

As used herein the term "polymerization retardant" means an agent that can stop or slow down the rate of polymerization. Examples of such agents are pure phosphoric acid, and 85% phosphoric acid. Certain opacifying agents, such as Pantopaque, Lipiodol and Ethiodol^{®} can also function as a polymerization retardant by diluting the amount of liquid monomer and hence slowing polymerization rate.

As used herein the term "a space" refers to an unfilled volume or cavity in a mass. Examples of such spaces, include but are not limited by the following, an existing space within a mass, such as, the lumen of a blood vessel, the sac of an aneurysm; a space created by a transiently placed external device, such as, a catheter or like device; a space created by a procedure, such as, an excision or like procedure; a space created by implantation of an object, such as, a stent or like device ; or a space created by the composition.

As used herein the term "stability" refers to the ability of a monomer component to resist degradation or polymerization after preparation but prior to use.

As used herein the term "inhibitor agent" refers to an agent which stabilizes a monomer composition by inhibiting polymerization. Within the context of the current invention, this term refers to agents that stabilize and inhibit polymerization by various mechanisms. By altering the amounts of one or more inhibitor agents, the rate of polymerization can be controlled. Inhibitor agents have different modes of activity, for example, hydroquinone acts primarily to inhibit high energy free radicals; *p*-methoxyphenol acts primarily to inhibit low energy free radicals; and phosphoric acid influences the rate of anionic polymerization.

As use herein the term "Neuracryl M" refers to the composition comprising of a monomer component ("M1") comprised of 2-hexyl cyanoacrylate, hydroquinone, *p*-methoxyphenol and phosphoric acid, and a second component ("M2") comprising of 2-hexyl cyanoacrylate monomer, ethyl myristate and gold. As noted above, the term "M1" refers to the monomer component of Neuracryl M, and the term "M2" refers to the second component of Neuracryl M.

As used herein the term "deployment device" refers a device used to deploy compositions, such as, those of the present invention. Examples of such devices, include but are not limited to the following. Micro Therapeutics, Inc., 2 Goodyear, Irvine, California 92618, markets medical devices, such as, the Rebar™ Micro Catheter, Equinox™ Occlusion Balloon System and SilverSpeed™ guidewires, that are used in conjunction for treating conditions such as those within the present invention. The devices disclosed in U.S. Patent No. 5,882,334 "Balloon/delivery Catheter Assembly with Adjustable Balloon Positioning," incorporated herein by reference, directed to a catheter assembly for delivering compositions.

### Nomenclature

The compound 2-hexyl cyanoacetate is depicted as follows, and also as Formula 3 in Schemes A and B.

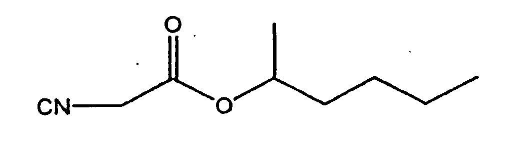

The compound 2-hexyl cyanoacrylate is depicted as follows, and also as Formula 5 in Scheme B.

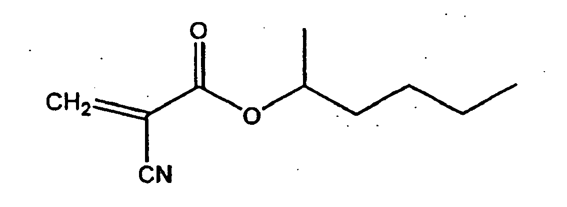

The present invention is a composition formed from alkyl cyanoacrylate monomeric units, such as, *n*-butyl, isobutyl, and 2-hexyl cyanoacrylate with at least one inhibitor agent, such as hydroquinone, , *p*-methoxyphenol and phosphoric acid. The composition polimerizes when it comes in contact with an non-ionic environment, such as, blood or tissue. The polimerized composition has characteristics which makes it particularly well suited as an embolic agent.

The composition of the present invention possess the following properties, which are desirable in an embolization agent.
1) The composition can be prepared and maintained as a monomeric component and second component until needed.
2) The composition has the ability to reliably and predictably change from a liquid state to a solid state, which is essential for its introduction and controlled placement into the lumen of vessel, duct, fistula or other like body passageways.
3) The composition has low viscosity, which is essential for its administration by syringes and micro-catheters or other like devices.
4) The composition has cohesive characteristics such that when the composition in administered into an non-ionic fluid environment, such as blood, the composition forms a single aggregate structure.
5) The composition has adhesive characteristic such that it attaches to the lumen of vessel, duct, fistula or other like body passageways, but not to the degree where the device depositing the composition will become fixed to it before the practitioner can remove it.
6) The composition causes mild tissue inflammation, sufficient to cause scarring, but not so severe to cause the formation of pus. Scar formation is necessary to maintain the functionality of the embolic block after the composition has biodegraded, or otherwise eliminated from the lumen.
7) The composition is sufficiently stable to biodegradation to allow for scarring to occur.
8) The composition is radiopaque. Although not necessary for its function as an embolic agent, radiopacity allows the embolic block to be observed with x-ray or other such imaging techniques.
9) The rate of heat released during polymerization of the composition is low enough such that the heat does not adversely effect surrounding tissues that may be heat sensitive, such as brain tissue.
10) The composition and its biodegradation products are sufficiently non-histotoxic and non-cytotoxic so that its presence is well tolerated in the body.

The composition of the present invention is used by combining the monomer component and second component. Upon mixing of the components, the invention is administered into the lumen of a blood vessel, duct, fistula or other like body passageways. The characteristics of the present invention permit its accurate placement in the lumen. Contact with an non-ionic environment, such as blood, or tissue causes the composition to polymerize. The size of the resultant embolic block formed is determined by the amount of composition administered.

The characteristics of the composition of the invention can be modified for a specific purpose or environment for which the embolic agent is intended to be utilized. For example, changes in the length and isomeric configuration of the alkyl side chains can alter the brittleness of the polymerized cyanoacrylate monomers. Alkyl chains that result in the formation of smaller aggregates tend to be less brittle, while larger aggregates tend to be less flexible. In addition, by combining monomers with different alkyl side chains the characteristics of the resultant polymer can be modified to what is optimal for a desired application.

Cyanoacrylates generate heat as they change from monomeric to polymeric form. The amount and rate of heat released, if excessive, can have a detrimental effect on the living tissue proximate to the vessel. Control of the amount and rate at which heat is release during polymerization is critical to the utility of composition.

### Preparation of the Monomer Component

The monomer component of the present invention is prepared by forming the desired precursor ester from the corresponding alkyl alcohol and cyanoacetic acid resulting in the desired alkyl cyanoacetate as depicted in Scheme A. The starting materials for this reaction are commercially available, for example from Aldrich Chemical Company, Sigma Chemical Company or Fluka Chemical Company, or can be prepared following procedures known to those of ordinary skill in the art.

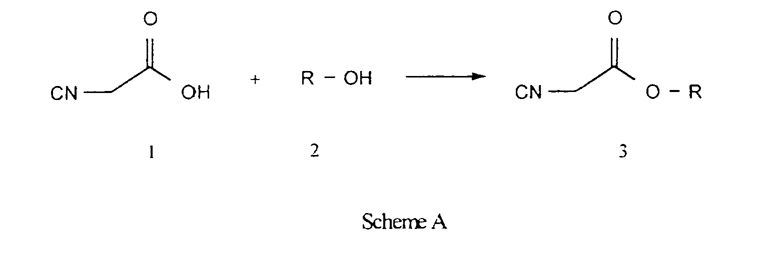

The compound of Formula 2 can be any alkyl alcohol, where R is from one to sixteen carbons, including but not limited to alcohols based on alkyl groups, such as, methyl, ethyl, propyl, butyl, pentyl, hexyl, heptyl, heptyl, octyl, nonyl, deca, undeca, dodeca, trideca, tetradeca, pentadeca and hexadeca, where the preceding moieties are linear (e.g., *n*-propyl, *n*-butyl, *n*-pentyl) or variously branched, such as *sec*-butyl, iso-butyl, *tert*-butyl, *iso-*propyl, 2-butyl, 2-pentyl, 2-hexyl, 2-heptyl, 2-octyl and the like. Particularly advantageous alcohols are those disclosed in U.S. Patent 3,728,375 entitled "Cyanoacrylate Adhesive Compositions", which is hereby incorporated by reference. Especially preferred are n-butyl, iso-butyl and 2-hexyl alcohols.

About 1 molar equivalents of the compounds of Formula 1 and Formula 2 are combined in a solvent like toluene at about 100 ml/molar equivalents. To this mixture is added a catalytic amount (about 1.0 x 10⁻⁴ molar equivalents) of *p*-toluene sulfonic acid. The mixture is stirred and heated to reflux. The preparation ideally yields the desired alkyl cyanoacetate at a purity level of about 95%. The experimental conditions can be readily modified by one of ordinary skill in the art without deviating from the present invention. Aspects such as, solvent selection, reaction time, temperature and choice of reagents are well within the skill of one of ordinary skill in the art. If necessary, the material can be further purified using multiple distillations and purification techniques and procedures known to those of ordinary skill in the art, such as water extraction, vacuum distillation, column chromatography, and the like.

### Preparation of alkyl cyanoacrylate

The desired alkyl cyanoacrylate monomer component of the present invention is synthesized from the alkyl cyanoacetate by reacting the it in a Knöevengel type reaction as depicted in Scheme B. 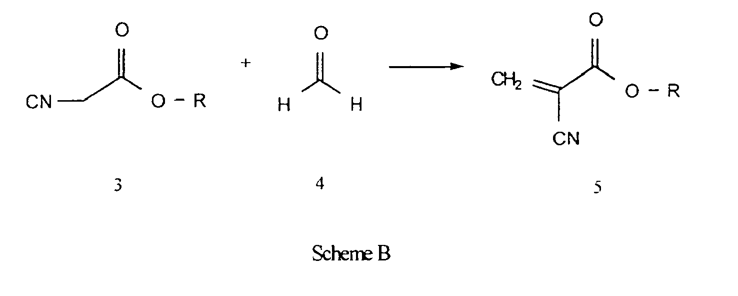 About 1 molar equivalents of formaldehyde (Formula 4), which is prepared from paraformaldehyde, and piperidine (at about 0.33 ml/molar equivalents) are combined in a solvent, such as methanol (at about 166 ml/molar equivalents). To this mixture is added about 1 molar equivalents of previously prepared alkyl cyanoacetate (Formula 3) in a dropwise manner. The reaction mixture is refluxed with stirring yielding the desired alkyl cyanoacrylate polymer (Formula 5). The reaction mixture is further processed with about 0.2 to 0.7 molar equivalents, preferably about 0.2 to 0.6 molar equivalents of phosphorous pentoxide yielding the desired alkyl cyanoacrylate. Care must be taken during purification steps to prevent the compound of Formula 5 from polymerizing. To this end the system is treated with trace amounts of sulfur dioxide, and receiver flasks are treated with hydroquinone and 85% phosphoric acid. After initial purification, the desired alkyl cyanoacrylate is further purified using multiple distillations, or other purification techniques known to those of ordinary skill in the art, such as, vacuum distillation, spinning band column, and the like.

### Formulation

The monomer component of the present invention comprises of at least one alkyl cyanoacrylate and at least one inhibitor agent. Typical inhibitors appropriate for cyanoacrylates are, for example, hydroquinone, *p*-methoxyphenol, pure phosphoric acid, and alkyl carboxylic acids, where the alkyl moiety ranges from 1 carbon, e.g., acetic acid, through to 15 and 17 carbons atoms in length, i.e., palmitic and stearic acids, respectively; and phosphoric acid at varying percentage solutions. Preferably hydroquinone, *p*-methoxyphenol, and phosphoric acid are used, individually or in combination.

Different inhibitors have different physical characteristics and thereby functions to alter the final properties of the composition. For example, hydroquinone is primarily an inhibitor for high energy free radicals; *p*-methoxyphenol is primarily an inhibitor for low energy free radicals; and phosphoric acid acts to control or inhibit anionic polymerization and the rate of such polymerization.

The quantity of inhibitors used is measured in terms of parts per million of alkyl cyanoacrylate. For example, for 2-hexyl cyanoacrylate, hydroquinone is in the range of about 50 to 150 parts per million (PPM), *p*-methoxyphenol in the range of about 50 to 150 PPM, and phosphoric acid in the range of about 125 to 375 PPM, more preferred is hydroquinone in the range of about 75 to 125 PPM, *p*-methoxyphenol in the range of about 75 to 125 PPM, and phosphoric acid in the range of about 187.5 to 312.5 PPM, and most preferred is hydroquinone in the range of about 95 to 105 PPM, *p*-methoxyphenol in the range of about 95 to 105 PPM, and phosphoric acid in the range of about 200 to 300 PPM.

The second component functions as an opacifying agent and a polymerization retardant.' To this end, the second component can be an iodinated oil, such as Ethiodol^{®}, or a brominated oil. Typically the iodinated oil is mixed as some percent of the total volume of the final composition. The percentage solution of iodinated oil used will influence the polymerization rate and opacity of the composition. Generally advantageous ranges are from about 17% to 66%, preferably about 33%.

Alternatively, the second component can be a composition comprising, a opacifying material, such as gold, platinum, tantalum, titanium, tungsten and barium sulfate and the like; an alkyl cyanoacrylate polymer material, and an alkyl esterified fatty acid, where the fatty acids have 3, for example, alkyl butyrate to 17 carbons, for example, alkyl stearate, preferred are, alkyl laurate, alkyl myristate, alkyl palmatate, and alkyl stearate, most preferred is alkyl myristate, and most especially prefereed is ethyl-myristate.

The opacifying material is used in a fine powder form, typically, with individual particles sized no larger than about 7 µm in diameter, preferably about 5 µm, most preferred about 2 µm and most especially preferred is 1 µm or smaller.

The amount of opacifying material used relative to alkyl cyanoacrylate polymer will vary according to the specific materials. Factors that influence the determination of the ratio include the amount and size of the particles that are being coated by the alkyl cyanoacrylate polymer. For example, for 2-hexyl cyanoacrylate and gold, 2 g of 2-hexyl cyanoacrylate is used per 100 g of powdered gold (particle size of about 5 ± 2 µm) being coated. The amounts vary accordingly with the opacifying material being coated by the alkyl cyanoacrylate. The alkyl cyanoacrylate and opacifying material are mechanically mixed by processing the alkyl cyanoacrylate into small particulate masses, and mixing with the finely powdered opacifying material. The alkyl cyanoacrylate polymer coated material is then stored in an esterified fatty acid, which serves as a medium where the alkyl cyanoacrylate polymer coated material is maintained prior to use, and as a medium, which when contacted with the monomer component will not interfere with the polymerization of the composition. The unsealed storage containers, preferably appropriately sterilized bottles and caps or the like, with the cyanoacrylate polymer suspension is then treated with ethylene oxide, or alternatively ketene. This treatment should occur no later than about 48 hours after completion of the coating process, preferably within 24 hours. The treatment process provides sterilization and stabilization of the alkyl cyanoacrylate polymer coated material and follows standard procedures for ethylene oxide use, i.e., positioning the containers so that they are amply exposed to the gas for a sufficient period of time.

### Polymer M

The characteristics of the composition of the invention can be modified for a specific application or environment in which the composition is intended to be utilized. For example, changes in the length and isomeric configuration of the alkyl side chains can alter the brittleness of a polymer formed from a cyanoacrylate monomer. Alkyl chains that result in the formation of smaller aggregates tend to be less brittle, while larger aggregates tend to be less flexible. Another method of modifying the characteristics of a polymer is to use a composition comprising of two or more types of alkyl cyanoacrylate monomers in combination with the appropriate inhibitors.

For example, a composition comprised of a monomer component comprising of 2-hexyl cyanoacrylate, hydroquinone, *p*-methoxyphenol and phosphoric acid; and a second component comprising of 2-hexyl cyanoacrylate polymer, gold, and ethyl myristate results in Polymer M.

A qualitative survey of Polymer M is shown in Table A. The physical characteristics disclosed are readily recognized by those of ordinary skill in the art as being relevant to the applications for which the polymers are used.

**Table A**

| Characteristics | Polymer M |
|---|---|
| cohesion | excellent |
| adhesion | moderate - low |
| polymerization profile | polymerizes to semi-solid to soft-solid on contact with tissue or blood |
| tactile | rubbery/gummy |
| molecular weight | low (1500 to 3000) |
| inflammatory response | mild response |
| viscosity | 14 to 15 centipoise |
| radio opacity | yes |
| exothermicity | low |

Polymer M has excellent cohesion properties. When introduced into a stationary fluid, or a fluid stream in motion, such as, the lumen of a blood vessel or other like passageway, the composition tend to stick together to itself remaining intact as a single mass or aggregate. This permits the composition to be discretely deployed or placed at the desired location without the hazard of having potions of the composition breaking away and depositing at undesired locales. Polymer M has viscosity properties that permit the injection of the liquid composition into a lumen of a blood vessel, duct, fistula or passageway in the body without using excessive pressure.

With these properties Polymer M is ideally suited for applications where the composition must penetrate further into non-ionic environment before arriving at the point of final placement. A preferred use is the treatment of arteriovenous malformations, also known as "AVM". Polymer M is also ideally suited for the treatment of longer type urinary fistulas, this is because preferred treatment requires greater penetration into cavity space by the liquid composition. Additional applications suited for Polymer M are creating a tubal occlusion, and surgical adhesions. For example, a composition of the present invention is applied to raw intraperitoneal tissue to prevent the tissue from adhering to itself or other tissue.

### Administration

The composition of the present invention are administered with any type of commercially available needle, catheter devices, or stereotaxic placement devices, preferably in conjunction with imaging technology that provides the practitioner with guidance as to the placement of the composition. Such devices and methods are readily known to those of ordinary skill in art. For example in U.S. Patent 5,925,683 "Liquid Embolic Agents", herein incorporated by reference, there is disclosed a method for introducing liquid embolic agents/solutions into the human body to form precipitated embolic occlusion masses, and also how this method is used for treating hepatic tumors using portal vein embolism. In U.S. Patent 5, 702, 361 "Method for Embolizing Blood Vessels", herein incorporated by reference, there is disclosed a method of embolizing a vascular site in a patient's blood vessel comprising of introducing, via a catheter, at the vascular site to be emobolized a non-particulate agent or a plurality of such agents, and delivering, via a catheter, to said vascular site a polymer composition comprising a biocompatible polymer, a biocompatible solvent and contrast agent, wherein the delivery is conducted under conditions where the polymer precipitate forms in situ at the vascular site resulting in the embolizing of the blood vessel and where the non-particulate agent is encapsulated within the precipitate. Additional devices applicable to the present invention are those disclosed in U.S. Patent No. 5,882,334 "Balloon/delivery Catheter Assembly with Adjustable Balloon Positioning," incorporated herein by reference, directed to a catheter assembly for delivering compositions. Further, Micro Therapeutics, Inc., 2 Goodyear, Irvine, California 92618, markets medical devices, such as, the Rebar™ Micro Catheter, Equinox™ Occlusion Balloon System and SilverSpeed™ guidewires, that are approved by the U.S. Food and Drug Administration for use in treating conditions such as those within the present invention.

The compositions of the present invention can be used advantageously in conjunction with any embolization method that employs an embolizing agent, occluding agent, or such composition that creates an embolic block, or occlusion.

The compositions of the present are used to particular advantage in conjunction with commercially available stereotaxic devices which facilitate the precise deposition of the composition, such as, for forming an occlusion within a cavity that is to be filled, or for forming an occlusion in a blood vessel that is the source of blood supply for a tumor.

### Utility

The present invention is useful for filling, occluding, partially filling or partially occluding an unfilled volume or space in a mass (*"*a space*"*). In particular, the composition is useful for filling an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition; the composition is also useful for adhering tissue to tissue, or adhering tissue to a device. The composition has the property of polymerizing when it comes in contact with an non-ionic environment, or when it is deployed in situ in an existing space, e.g., the lumen of a blood vessel, or the sac of an aneurysm, a space created by a transiently placed external device, e.g., a catheter or like device, a space created by a procedure, e.g., an excision or like procedure or implantation of an object, e.g., a stent or like device, or a space created by the composition.

The present invention is useful as an embolic agent that selectively creates an embolic blockage in the lumen of a blood vessel, duct, fistula or other like body passageways.

The present invention can be prepared and maintained as a monomeric component and second component until needed. It has the ability to reliably and predictably change from a liquid state to a solid state, which is essential for its introduction and controlled placement into the lumen of vessel, duct, fistula or other like body passageways. The composition has low viscosity, which is essential for its administration by syringes and micro-catheters or other like devices.

The cohesive characteristics of the invention are such that when the composition in administered into an non-ionic fluid environment, such as blood, the composition forms a single aggregate structure. Additionally, the adhesive characteristics are such that the composition attaches to the lumen of vessel, duct, fistula or other like body passageways, but not to the degree where the device depositing the composition will become fixed to it before the practitioner can remove it.

The present invention causes mild tissue inflammation, sufficient to cause scarring, but not so severe to cause the formation of pus. Scar formation is desirable as the scar tissue is necessary to maintain the functionality of the embolic block after the composition has biodegraded, or otherwise eliminated from the lumen. The composition is sufficiently stable to biodegradation to allow for scarring to occur.

The present invention is radiopaque. Although this characteristic is not necessary for its function as an embolic agent, radiopacity allows the embolic block to be observed with x-ray or other such imaging techniques.

The rate of heat released during polymerization of the present invention is low enough such that the heat does not adversely effect surrounding tissues that may be heat sensitive, such as brain tissue.

The present invention and its biodegradation products are sufficiently non-histotoxic and non-cytotoxic so that its presence is well tolerated in the body.

The composition of the present invention is useful for filling, occluding, partially filling or partially occluding an unfilled volume or space in a mass (*"*a space*"*).

The present invention describes a method for filling, occluding, partially filling or partially occluding an unfilled volume or space in a mass. The types of unfilled volumes or spaces within the scope of the present invention includes, but are not limited to the following instances.

For example, the present invention is used as a method of filling, occluding, partially filling or partially occluding an existing space, such as, a lumen of a passageway in the body, e.g., a blood vessel, a duct, an aneurysm, or a fistula. Examples of the types treatments covered by this method of use, include but are not limited to the following. The present invention is useful as a method of treating arteriovenous malformations (AVM) where the blood vessel(s) that feed the AVM are occluded thereby cutting off the blood supply to the AVM. The present invention is useful as a method to ablate diseased or undesired tissue by cutting off the tissue's blood supply. In particular, the present invention is useful as a method of treating a tumor having a discrete blood supply, where the blood vessel(s) that feed the tumor are occluded thereby cutting off the blood supply to the tumor resulting in diminished growth or death of the tumor. The present invention is useful as a method of preventing or mitigating the development of an aneurysm by creating a partial occlusion at a location in the blood vessel selected to modify the fluid dynamics within the vessel to mitigate the formation or development of an aneurysm. The present invention is useful as a non-surgical method of treating symptomatic uterine leiomyomas by embolizing/occluding the uterine artery. This method has been reported using a non alkyl cyanoacrylate composition in the Journal of Vascular and interventional Radiology, 10:891-894, July-August 1999. The present invention is useful as a method of sterilizing a female mammal by occluding the fallopian tubes thereby preventing the passage of the eggs from the ovaries to the uterus. The use of an occluding agent to sterilize a female mammal is disclosed in U.S. Patent No. 5,989,580 *"*Method of Sterilizing Female Mammals,*"* herein incorporated by reference. The methods disclosed in this patent can be advantageously applied using the compositions of the present invention, and are within the scope of the present invention.

The present invention is an embolic agent that provides a method for selectively creating and placing an embolic blockage which mechanically blocks, totally or partially, the lumen of a blood vessel, duct, fistula or other body passageway. In particular, the current invention is particularly useful in blocking, totally or partially, or diverting the flow of blood through the lumen.

The present invention can be advantageously used to block blood flow to certain tissues or areas. For example, the present invention can be used to treat arteriovenous malformation (AVM). An AVM is a collection of abnormal blood vessels which are neither arteries or veins. These vessels are packed closely together to form the nidus of the AVM. Blood flow into the AVM nidus is through thinned, enlarged, tortuous vessels and is rapidly shunted into draining veins because the nidus contains no arterioles or capillaries to provide high resistance. Clinical symptoms experienced because of AVMs are bleeding, re-direction of blood from nearby normal structures, or seizures. The primary clinical problem associated with cerebral AVM is the potential for lethal hemorrhage. The current standard of care for treating AVMs is surgical removal, high energy radiation or embolization with particular devices.

Further, the present invention can be used for treating cancer by diverting or blocking blood flow to tumors, the present invention is particularly useful for treating tumors in areas that are not easily accessible for surgical intervention, for example, brain tumors.

Other advantageous uses of the present invention are for aortopulmonary closure; treatment of artery pseudoaneursym; hepatic artery vascular occlusion and for temporary vascular occlusion during coadministration of cytotoxic drugs; treatment of other types of vessels, for example, the composition can be used for creating tubal occlusions, fallopian tube occlusions, vas deferens occlusions, and urinary occlusions.

The present invention provides a method of filling, occluding, partially filling or partially occluding a space created by a transiently placed external device, such as, a catheter balloon. Examples of the types of treatments covered by this method of use include, but are not limited to the following. The present invention is useful as a method of treating an aneurysm by filling the space within the aneurysm with a composition of the present invention, where the composition polymerizes in the space within the aneurysm, thereby preventing the rupture of the aneurysm. This treatment can be effected using the present invention with any number of catheters, catheter coils, catheter wires or catheter balloons commercially available. Examples of such devices are commercially available from sources. For instance, Micro Therapeutics, Inc., 2 Goodyear, Irvine, California 92618, markets a line of medical devices, such as, the Rebar™ Micro Catheter, Equinox™ Occlusion Balloon System and SilverSpeed™ guidewires. Similarly, U.S. Patent No. 5,882,334 "Balloon/delivery Catheter Assembly with Adjustable Balloon Positioning," assigned to Target Therapeutics, Inc., and incorporated herein by reference, is directed to a catheter assembly for delivering compositions, such as, those of the present invention.

The present invention also provides a method of filling, occluding, partially filling or partially occluding a space created or resulting from a procedure, such as with the excision of tissue, or insufflation. Examples of the types of treatments covered by this method of use include, but are not limited to the following. The present invention is useful as a method of treating oozing capillaries following an excision procedure.

The present invention further provides a method of filling, occluding, partially filling or partially occluding a space created by the placement or implantation of an object, such as, a medical device. Examples of the types of uses covered by this method of use include, but are not limited to the following. The present invention is useful as a method of restoring the normal fluid dynamics at the peripheral edges of a vascular stent by filling the dead spaces between the stent and the lumen wall created by the implantation of the stent.

Still another advantageous use is the controlling and smoothing the blood flow around stents. A major complication from the balloon angioplasty and the use of stents is disruption of the smooth flow of blood past and around the stent which can lead to the formation of blood clots and their associated complications. The composition of the present invention can be used to modify and make regular the slip streams of blood through and adjacent to the stent to mitigate or alleviate the cause of the turbulence, and such turbulence causing states.

The present invention further provides a method of filling, occluding, partially filling or partially occluding a space created by the composition itself, such as, where the composition is used as a bulking agent. Examples of the types of uses covered by this method of use include, but are not limited to the following. For example, a method of recreating normal external contours, such as following physical trauma.

### Administration

The monomer component and second component of the present invention are combined just prior to use. The composition of the present invention is administered using any type of deployment device. The term "deployment device" refers to a device used to deploy fluids or compositions similar to those of the present invention, such as, a needle, catheter devices, catheter balloon, stereotaxic placement devices, or the like. Methods for using these devices are readily known to one of ordinary skill in the art, and such devices are commercially available. Such devices and methods are readily known to those of ordinary skill in art. For example in U.S. Patent 5,925,683 "Liquid Embolic Agents", herein incorporated by reference, there is disclosed a method for introducing liquid embolic agents/solutions into the human body to form precipitated embolic occlusion masses, and also how this method is used for treating hepatic tumors using portal vein embolism. In U.S. Patent 5,702,361 "Method for Embolizing Blood Vessels", herein incorporated by reference, there is disclosed a method of embolizing a vascular site in a patient's blood vessel comprising of introducing, via a catheter, at the vascular site to be emobolized a non-particulate agent or a plurality of such agents, and delivering, via a catheter, to said vascular site a polymer composition comprising a biocompatible polymer, a biocompatible solvent and contrast agent, wherein the delivery is conducted under conditions where the polymer precipitate forms in situ at the vascular site resulting in the embolizing of the blood vessel and where the non-particulate agent is encapsulated within the precipitate. Additional devices applicable to the present invention are those disclosed in U.S. Patent No. 5,882,334 "Balloon/delivery Catheter Assembly with Adjustable Balloon Positioning," incorporated herein by reference, directed to a catheter assembly for delivering compositions. Further, Micro Therapeutics, Inc., 2 Goodyear, Irvine, California 92618, markets medical devices, such as, the Rebar™ Micro Catheter, Equinox™ Occlusion Balloon System and SilverSpeed™ guidewires, that are approved by the U.S. Food and Drug Administration for use in treating conditions such as those within the present invention.

The composition of the present invention are administered with any type of commercially available needle, catheter devices, or stereotaxic placement devices, preferably in conjunction with imaging technology that provides the practitioner with guidance as to the placement of the composition. The compositions of the present invention can be used advantageously in conjunction with any embolization method that employs an embolizing agent, occluding agent, or such composition that creates an embolic block, or occlusion, or otherwise in effect is used for filling, occluding, partially filling or partially occluding an unfilled volume or space in a mass ("a space").

### EXAMPLES

The following examples are given to enable those of ordinary skill in the art to more clearly understand and to practice the present invention. The examples should not be considered as limiting the scope of the invention, but merely as illustrative and representative thereof.

### EXAMPLE 1

### PREPARATION OF 2-HEXYL CYANOACETATE

A 5 liter, 24/40 ground glass jointed flask was configured with a reflux condenser, Dean-Stark trap, and football magnetic stirring bar. The reaction vessel was charged with the 1,275.0 g of cyanoacetic acid (Aldrich Chemical Co.), 1,581.5 g of 2-hexanol (Aldrich Chemical Co.) and 3.0 g of p*-*toluenesulfonic acid (Aldrich Chemical Co.), and 1,500 of toluene (Aldrich Chemical Co.). The reaction mixture was stirred and heated to reflux. Water was formed as a byproduct of the reaction and was collected during the course of the reaction. The reaction was continued until there was a period of over 30 minutes where no water was produced. The amount of water collected was 230 ml and indicated that the reaction had completed with a 85.2% theoretical yield. The reaction mixture was allowed to cool to room temperature.

The reaction mixture was stirred and 500 ml of a saturated baking soda (sodium bicarbonate) solution was gradually added to the mixture. The reaction mixture was stirred vigorously until the frothing stopped. The reaction mixture was poured into a six liter separatory funnel, to which an additional 500 ml of water was added. The funnel was vigorously agitated. The aqueous phase was separated and saved as Reaction Water. The pH of the aqueous layer was measured to insure that the pH was over 8. Another 500 ml of water was added to the organic phase reaction mixture in the separatory funnel. The contents of the funnel were again agitated, the aqueous and organic phases were allowed to settle, and the aqueous phase separated and also saved as Reaction Water. This washing procedure was repeated two additional times. The organic phase was moved to a 5-liter flask. The flask was configured with a distillation condenser. The reaction mixture was heated to reflux, and the remaining water was separated from the mixture and discarded. The apparatus was reconfigured for vacuum fractional distillation. Initially, the toluene and 2-hexanol in the mixture were removed by reducing the pressure of the apparatus to about 5 Torr, and then by heating the mixture to 60° with stirring. After the solvents were removed, the pressure was further reduced to less than 1 Torr and gradually increased heat until the desired 2-hexyl cyanoacetate began to distill off. The heat was adjusted so that the product was recovered at a rate of 2 drops/sec. The recovery collected 1921.1 g (70.76% yield) of the 2-hexyl cyanoacetate, and was halted when no more material came out of the distillation unit. Gas chromatographic analysis of the purity of the 2-hexyl cyanoacetate indicated that the product was 98.3% pure, which was well above 95% purity requirement for proceeding to the next procedure.

If the purity of the 2-hexyl cyanoacetate had been below 95%, the material could have be purified by vacuum distillation, or using any like technique for purification known to those of ordinary skill in the art.

### EXAMPLE 2

### PREPARATION OF 2-HEXYL CYANOACRYLATE

A 5-liter three-necked flask was configured with a reflux condenser, Dean-Stark trap, an addition funnel and a mechanical stirrer with a glass paddle in a 5-liter heating mantle. Paraformaldehyde 272.4 g and methanol 1,500 ml were combined in the flask. The reaction mixture was heated to reflux and stirred for a period of 1 hr until the solution began to cleared. 3 ml of piperidine was washed into the reaction mixture with methanol, followed by 1521.9 g of 2-hexyl cyanoacetate added in a dropwise fashion. The resulting reaction was exothermic, and the heat was adjusted to maintain the reaction mixture at reflux temperature. The reaction mixture was refluxed for an additional 30 minutes after the conclusion of the addition. Methanol was distilled from the reaction mixture and collected through the Dean-Stark trap until 1420 ml of the original methanol (98%) was recovered (compensating for spillage). The reaction mixture was halted overnight at this point.

The reaction vessel was configured with a vacuum apparatus to collect residues, and placed under high vacuum to remove remaining volatile materials. The vacuum was gradually increased until less than 10 Torr was reached. The apparatus was heated until all the solvent had been removed. Once the solvent was removed, 75 g of phosphorous pentoxide was added to the mixture taking care to minimize its exposure to air. The heat was discontinued, and the mixture was stirred for one hour. The apparatus was then flooded with sulfur dioxide. The pressure was reduced to less than 10 Torr and heated slowly, the flow of sulfur dioxide was adjusted for a constant low-level flow of gas into the apparatus.

A 1 liter flask was washed with concentrated sulfuric acid, three times with water, and once with ultra pure water. The flask was oven dried for one hour at 110°C and was allowed to cool to room temperature. 10 drops of 85% phosphoric acid and approximately 25-50 mg of hydroquinone was added to the 1 liter flask. The flask was fitted as the receiver flask to the distillation apparatus. The pressure of the distillation was reduced to less than 10 Torr. The reaction mixture was heated and stirred until the distillation began. 418 g of 2-hexyl cyanoacrylate was collected at a 25% yield. The distillation was halted when the temperature rose above 110°C.

### EXAMPLE 3

### Purification of 2-Hexyl Cyanoacrylate

The 2-hexyl cyanoacrylate was purified in a two step process. The compound was first by vacuum distillation, and then further purified by spinning band column.

### Vacuum Distillation

A vacuum distillation apparatus was configured with a 2 L flask, magnetic stirrer, fraction cutter, a 10" Vigreux column a clasien head, condenser, thermometer and a 100 ml forecut receiving flask. 10 drops of 85% phosphoric acid and 10 mg of hydroquinone was added to the forecut flask. The unpurified 2-hexyl cyanoacrylate was place into the distillation flask and the pressure of the unit was reduced to just under 1 Torr. The material was stirred and gradually heated until product was being distilled and collected at a rate of one drop per minute. After 35 ml of distillate was collected, a second 2 L receiving flask that had been prepared by acid washing, followed by the addition of 25 drops of 85% phosphoric acid and 20 mg hydroquinone was placed to receive the distillate. The distillation rate was gradually increase till the product was being collected at a rate of 2-3 drops per second. When the distillation head temperature rose 2°C above that used to collect the main fraction, the distillation was completed. Heat was discontinued, and the material was allow to cool under dry air by air filtered through a drying tube.

### Spinning Band Column Purification

The spinning band column (B/R Instrument Corp., 9119 Centreville Road, Easton, Maryland 21601, Model 9600) is a long jacketed silvered column fitted with a 30/50 socket joint at the bottom. A magnetic stirring bar was added to the 5 L socket joint flask, which was then filled with the product to be purified. A heating mantle is supported on a magnetic stirrer that is raised and lowered with a laboratory jack to fit to the column. On the upper right hand side of the column there was another 30/50 male socket joint for a 100 ml receiving flask. All flasks and joints were greased with high vacuum grease to assure a good vacuum seal. When assembled, a glass temperature probe was inserted into the 10/15 joint of the flask, and a metal Tempora probe was inserted inside the glass probe. The 29/42 joint containing the stopcock was greased and placed into the flask and connected to a sulfur dioxide gas line. The pressure of the system was gradually reduced down to just under 1 Torr of pressure.

Operation of the spinning band column was controlled by a microprocessor. The column was programmed to operate under the following conditions, the water cooling temperature was set to 15°C, the column's motor turns on at 24°C, equilibration time was 2 min, open temperature 28°C, close temperature 90°C, mantle rate 24°C, reflux ratio 20 to 1 and pot temperature to end run 90°C. Just prior to beginning the distillation a small flow of sulfur dioxide was leaked into the system. The temperature of the flask was monitored during the distillation to ensure that the temperature at no time rose above 100°C. The distillate was collected in the receiver flask at the end of the distillation.

The contents in the flask of the spinning band column were allowed to cool for 30 min. A second high vacuum distillation apparatus configured identically to the vacuum distillation apparatus first used in this procedure was setup using a 2 L round bottom flask. To this flask was added 0.0269 g of hydroquinone, 0.0275 g of *p*-methoxyphenol, and 0.0794 g of phosphoric acid. The residue for pot of the spinning band column was added to the 2 L round bottom flask of the vacuum distillation apparatus. The contents of the flask was stirred and the pressure of the unit was reduced to just less than 1 Torr. A small stream of sulfur dioxide was leaked into the apparatus as the distillation continued. A receiver flask was prepared by adding 10 mg hydroquinone and 15 drops of 85% phosphoric acid. A forecut fraction of 86.3 g was collected at the rate of 2-6 drops per minute. The main fraction was collected in a receiver similarly prepared as the forecut fraction flask. 1620.1 g of main fraction product was collected at a rate of 20-25 drops per minute. The material was then re-distilled by the spinning band column under the previous conditions.

The purity of the 2-hexyl cyanoacrylate was determined by gas chromatography. The gas chromatograph was configured as follows,
HP 5890 Gas Chromatograph with HP Chemstation Software.
Column Description: Supelco Nukol (60 meter, I.D.-0.32 mm, film thickness-1 micron).

| Instrument Parameters: | Method 1 |
|---|---|
| Injector Temperature: | 220°C |
| Detector Temperature: | 280°C |
| Head Pressure: | 15 PSI |
| Air Pressure: | 35 PSI |
| Hydrogen Pressure: | 20 PSI |
| Aux: | 60 PSI |
| Initial Oven Temperature: | 140°C for 20 min. |
| Ramp: | 5°C/min. |
| Final Oven Temperature: | 200°C for 50 min. |
| A Splitless System. | |
| Injection Volume: | 1.0 micro liter |

1.0069 g of the 2-hexyl cyanoacrylate was mixed thoroughly with 2 drops of 1-hexanol (0.0044 g), was analyzed and impurities were found to be at an acceptable for use. The 2-hexyl cyanoacrylate was sufficiently pure to use for product.

### EXAMPLE 4

### FORMULATION OF THE MONOMER COMPONENT

The monomer component was formulated with the following materials 2-hexyl cyanoacrylate 1249.9 g, hydroquinone 0.0764 g, *p*-methoxyphenol 0.0874 g and phosphoric acid 0.1693 g. The hydroquinone and *p*-methoxyphenol were kept under reduced pressure in a dessicator over a drying agent. The pure phosphoric acid was particularly deliquescent and care was taken not permit water contamination. The calculated molar quantities and PPM of each material were as follows,

| Material | Moles | PPM |
|---|---|---|
| 2-hexyl cyanoacrylate | 6.8964 | 999,547 |
| hydroquinone | 0.000694 | 100 |
| *p*-methoxyphenol | 0.000704 | 102 |
| phosphoric acid | 0.001726 | 250 |

Overall purity of the formulation was determined by gas chromatograph to be less than 1%, using 1-hexanol as an internal standard.
Instrument Description: HP5890 Gas Chromatograph with HP chemstation software.
Column Description: Supelco Nukol (60 meters-length, I.D., 0.32 mm, Film Thickness 1 micron)

| Instrument Parameters: | Method 1 |
|---|---|
| Injector Temperature: | 220°C |
| Detector Temperature: | 280°C |
| Head Pressure: | 15 PSI |
| Air Pressure: | 35 PSI |
| Hydrogen Pressure: | 40 PSI |
| Aux.: | 60 PSI |
| Initial Oven Temperature: | 140°C for 20 min. |
| Ramp: | 5°C/min. |
| Final Oven Temperature: | 200°C for 50 min. |
| A Splitless System: | |
| Injection Volume: | 1.0 microliter |

### EXAMPLE 5

### PREPARATION OF THE 2-HEXYL CYANOACRYLATE POLYMER COMPONENT ("the Second Component")

Ethyl myristate was obtained commercially from Aldrich Chemical Company at 97% purity. Prior to use, the ethyl myristate was further purified by vacuum distillation to 99.8% purity following standard routine chemical procedures.

Six 3 ml syringes were fill with purified 2-hexyl cyanoacrylate. 500 mg of sodium bicarbonate and 250 ml of ultra pure water were placed into a Waring blender. The lid of the blender was adjusted so that the contents of the syringes could be emptied dropwise into the center of blender while the blender was stirring. With the speed of the blender set to high, each of the syringes were emptied in a dropwise fashion into the stirring blender. When the addition was completed, the lid of the blender was closed and the mixture was stirred for another minute. The solution was decanted from the blender leaving just solid material in the blender. Residual solid material that was inadvertently removed with the decanted solution was recovered by filtration, washed with ultra pure water and placed back into the blender. Solid material adhering to the inside portion of the blender was rinsed with ultra pure water back with the rest of the solids in the blender. An additional 250 ml of ultra pure water was added into the blender, and the water and solid mixture was blended for 1 minute. Following the last procedure, water solution was decanted through a large coarse fritted glass funnel filter that recovered solid material in the solution. The solid material was washed with methanol prior to be added back to the rest of the solid material. The walls of the blender were rinsed with methanol to collect all the solid material back into the blender. 250 ml of Methanol was added to the blender. The solids were blended for one minute. The solid material is filtered from the methanol. Any residual solid material in the blender is washed with methanol and combined with the solid material filtered from the methanol. The solid material on the filter was placed under a low vacuum to remove the rest of the methanol. The solid material was moved quantitatively to a 100 ml round bottom standard tapered flask. The flask was placed under reduced pressure to remove the remaining methanol. 2 g of the solid material was combined with 100 g of powdered gold. The mixture was placed into a standard laboratory blender and blended for one minute. The blender was agitated constantly during the blending to ensure that the gold did not settle during the blending. 1.020 g portions of the blended material were placed into previously cleaned and prepared bottles. To each bottle was added 500 mg of ethyl myristate at 99.8% purity. The filled bottles were kept under a Laminar flow hood. The unsealed bottles were arranged in trays for immediate ethylene oxide sterilization by Sharp Coronado Hospital, Sterile Processing Department under standard conditions.

### EXAMPLE 6

### COMPARISON OF CATHETHER ADHESION FORCE FOR 2-HEXYL CYANOACRYLATE (NEURACRYL M) AND n-BUTYL CYANOACRYLATE (HISTOACRYL) COMPOSITIONS

The present invention is also known by the name of Neuracryl M, where Neuracryl M1 corresponds to the monomer component, and Neuracryl M2 corresponds to the second component comprising of the gold coated 2-hexyl acrylate. This example demonstrates differences in adhesion between the two cyanoacrylates by measuring the amount of force required to remove a catheter from various compositions of Neuracryl and Histoacryl. Histoacryl is commercially available from Braun GmbH. It is similar to Neuracryl M in that it is a polymer composition also based on a cyanoacrylate structure, i.e., n-butyl cyanoacrylate. However, the force required to withdraw a catheter from Histoacryl is greater than that required for Neuracryl M, and in this key aspect, Neuracryl M possesses a surprising and unexpected advantage over Histoacryl.

The force resulting from catheter adhesion was determined for Neuracryl M1 and M2 (mixed), pure Neuracryl M1, Neuracryl M1 mixed with 33% Ethiodol^{®}, Neuracryl M1 mixed with 50% Ethiodol^{®}, pure Histoacryl, Histoacryl mixed with 33% Ethiodol^{®}, and Histoacryl mixed with 50% Ethiodol^{®} were measured and compared.

All the mixtures were injected through a TurboTracker micro catheter device (Medi-tech/Boston Scientific, Watertown, MA). All mixtures were prepared immediately prior to use to prevent separation of the components or contamination. The catheter tips were placed at the bottom of 10 mm deep by 5 mm diameter wells filled with 0.2 mL of heparinized human whole blood. Through the micro catheter, 0.15 mL of each embolic mixture was injected into each well, surrounding the tip of the micro catheter. Mixtures containing Histoacryl were allowed to polymerize for 1 minute, and those containing Neuracryl for 3 minutes. The microcatheters were then extracted from the polymerized cyanoacrylates at a constant speed of 8.3 mm/sec (Model 1000 Materials Testing System; Instron, Canton, MA) and the forces required for extraction were measured and recorded (Minibeam Force Transducer, 25-lb capacity; Interface Advanced Force Measurement, Scottsdale, AZ). Five samples of each mixture were tested. Comparison of the results was performed using the Student t test.

Successful mesurements of the peak forces required for the extraction of the catheters from the polymerized cyanoacrylates were obtained for six of the seven mixtures tested. A wide range of peak forces were required to extract the microcatheters from the various mixtures. The force of extraction for the Neuracryl M1 and 50% Ethiodol^{®} mixture was less than 0.05 Newtons and beyond the ability of the apparatus to obtain precise measurements. The peak forces required to extract the microcatheters from either Histoacryl mixed with 33% Ethiodol (1.44 N ± 0.33) were significantly higher (P<0.01; P<0.05) than those for pure Neuracryl M1 (1.00N ± 0.23). Histoacryl had to be mixed with 50% Ethiodol^{®} to decrease the force of extraction (0.34N ± 0.14) to less than that associated with pure Neuracryl M1 (P<0.01).

When Neuracryl M1 and M2 were mixed together the force required for micro catheter extraction (0.41N ± 0.14) was significantly lower than that for either pure Histoacryl (1.83 N ± 0.21), Histoacryl mixed with 33% Ethiodol^{®} (1.44 N ± 0.33), or Neuracryl M1 alone (1.00 N ± 0.23) (P<0.01; P<0.01; P<0.01, respectively). The force required to extract microcatheters from the Neuracryl M1 and M2 mixture was not, however, significantly different from that of Histoacryl mixed with 50% Ethiodol^{®} (0.41 N ± 0.14 vs. 0.34 N ± 0.14)

Although Neuracryl M1 was not designed to be mixed with Ethiodol^{®}, like Histbacryl, Neuracryl M1 demonstrated markedly decreased micro catheter adhesion when mixed with 33% and 50% Ethiodol^{®}. The extraction force was reduced significantly from 1.00 N ± 0.23 to 0.28 N ± 0.12 when Neuracryl M1 was mixed with 33% Ethiodol (P<0.01). There was no significant difference between the peak extraction forces for Neuracryl M1 mixed with 33% Ethiodol^{®} and Neuracryl M1 and M2 mixed was intended for clinical use. (0.28 N ± 0.12 vs 0.41 N ± 0.14). When Neuracryl M1 was mixed with 50% Ethiodol^{®}, the force of extraction was less than 0.05 N and below our limit for accurate measurement. The force was so low that, unlike with the other mixtures, no effacement of the slight natural curve of the catheter was observed prior to the tip of the catheter pulling out of the cyanoacrylate.

### EXAMPLE 7

### COMPARISON OF 2-HEXYL CYANOACRYLATE AND n-BUTYL CYANOACRYLATE INTERACTIONS WITH BLOOD AND IN AN ARTERIOVENOUS MALFORMATION MODEL

The following example compares the interaction of 2-hexyl cyanoacrylate composition of the present invention (2HCA) and a composition of 33% n-butyl cyanoacrylate and 66% ethiodol (NBCA), which is the clinical standard, with blood.

2HCA was compared to NBCA in heparinized pig blood under four conditions:
(1) a drop of each composition was placed on the surface of blood, observed, and the polymerization process was timed.
(2) a 22 gauge needle was placed below the surface of static blood, and 0.4 milliliter of each composition was injected and observed over a 1 minute period;
(3) blood was circulated through a 4 millimeter I D polyvinyl chloride tubing at 40 centimeters per second. A 22 gauge needle inserted into the central slipstream introduced the compositions at rates varying from 0.1 ml to 8 ml per second powered by a Medrad mk 4 pressure injector. Behaviors were recorded via fluoroscopy on S VHS T V;
(4) standardized arteriovenous malformation models were placed in a circuit of pulsatile flowing blood, and the compositions were introduced via microcatheters under direct fluoroscopic control, using the same techniques used in humans. The models were later opened, the polymerized compositions were removed and their characteristics were compared. Polymer which escaped from the models were also collected downstream and examined.

### Findings:

(1) Dropping the compositions onto the surface of blood yielded generally equal polymerization times, about 2 seconds.
(2) When injected below the surface of static blood, the 2HCA formed a rubbery elastic mass which remained at the needle's tip. The NBCA compound fell away from the needle, to the bottom of the beaker and polymerized to a friable mass.
(3) When injected into blood flowing at physiologic velocities, the NBCA compound formed small, individual, nearly spherical droplets that did not remain as a cohesive mass, but rather broke away and embolized down stream. There was no injection rate at which we could keep the device from embolizing away, or to make it block the tube. Conversely, there was no rate of injection which could prevent the 2HCA from remaining as a cohesive polymerized mass and the tube was blocked solidly for the length of the injection.
(4) When injected into the AVM model, the 2HCA yielded significantly better penetration than the NBCA compound. The character of the polymerized compositions was significantly different: the NBCA compound made a firm yet friable mass much like dry cottage cheese; the 2HCA mass was elastic much like chewing gum.

In summary, the standard test of the cyanoacrylate drop on blood yielded no predictive information. However, when the cyanoacrylates were respectively injected below blood, strikingly different outcomes were observed. The NBCA immediately fell away from the needle to the bottom of the beaker, whereas the 2HCA remained as a cohesive whole at the needle tip. There was no introduction rate which could disrupt the cohesiveness of 2HCA; there was no introduction rate which allowed the NBCA composition to remain a cohesive whole. Particles of the NBCA composition formed and continually pushed downstream. Injection of 2HCA into standard AVM models yielded consistently better control penetration of the nidus of the AVM.

### EXAMPLE 15

### THE BEHAVIOR OF NEURACRYL M IN THE PIG RETE

This example compares the intravascular behavior of Neuracryl M to the current clinical standard, n-butyl cyanoacrylate 40% in the rete of the pig.

### Methods and Materials

Neuracryl M is available from Prohold Technologies, El Cajon, CA. Neuracryl M is a two-part embolization agent consisting of a glass ampule of 1.25 ml Neuracryl M1 and a rubber-stoppered glass vial of Neuracryl M2 (a mixture of 2-hexyl cyanoacrylate, an esterified fatty acid, and gold particles measuring approximately 5µm in diameter. Prior to use, the contents of the Neuracryl M1 vial are injected into the vial containing Neuracryl M2, and the two are shaken together thoroughly until mixed. The gold particles and esterified fatty acid are used to retard polymerization and provide radiopacity. To avoid separation of the components or contamination, the two moieties were not mixed until immediately before use. NBCA (Histacryl Blue) was obtained from B. Braun, Melsungen, Germany, and mixed with Ethiodol^{®} to form a 40% solution.

Nine barnyard laboratory pigs were placed under general anesthesia. Using sterile technique a catheter was placed in the right femoral artery and a guiding catheter in the left common artery. A microcatheter was introduced into the region of the rete, through which the entire cerebral blood passes.

With the pigs under general endotracheal anesthesia, using sterile technique, the right femoral artery was catheteried and a guiding catheter was placed into the left common carotid artery of nine pigs. A microcatheter was introduced into the region of the rete, and 0.1 to 0.3 of NBCA (*n*-butyl cyanoacrylate) or Neuracryl M was infused without flow arrest. After follow-up angiography, the opposite carotid and pharyngeal arteries were catheterized, and the alternative agent was infused. One pig was sacrificed immediately, six were sacrificed at 3 weeks, and two were sacrificed at 3 months. Data were assessed using a gross radiolegic method, the rete are radiographed and graded for degree of penetration by the composition being tested.

### Results

One pig was sacrificed immediately due to clinical infarction. Distal embolization of NBCA was found to be the cause.

In one pig (sacrificed immediately because of clinical infarction), distal embolization of the NBCA was found. In the other pigs, penetration of the embolic agent into the rete was graded as follows, 0, no penetration; 1, penetration of 25% or less; 2, 25-50% penetration; 3, 50-75% penetration; and 4, 75-100% penetration.

### Summary of the Results

| Material | Penetration mean | Catheter Trapped | Embolization Distal to Rete |
|---|---|---|---|
| Neuracryl M | 3.7 | 0/9 | 0/9 |
| NSCA | 1.8 | 4/9 | 2/9 (one death) |

Neuacryl M had a mean penetration grade of 3.7, compared to 1.8 for NBCA. Trapping of the catheter by the glue mass occurred in four of nine pigs with NBCA, whereas there were no occurrences in the pigs treated with Neuracryl M. Embolization occurring distal to the rete occurred in two pigs infused with NBCA, with one resultant death. There were no occurrences of embolization occurring distal to the rete in pigs infused with Neuracryl M.

| Inflammation at 3 weeks (6 pigs) | | | | | |
|---|---|---|---|---|---|
| Material | Intraluminal | Mural | Perivascular | Interstitial | Giant Cells |
| Neuracryl M | 1.9 | 2.2 | 2.0 | 1.0 | 5/7 |
| NBCA | 0.6 | 0.8 | 0.8 | 0.2 | 2/7 |

| Inflammation at 3 months (2 pigs) | | | | | |
|---|---|---|---|---|---|
| Neuracryl M | 3.0 | 3.0 | 3.0 | 0.3 | 2/2 |
| NBCA | 1.5 | 1.5 | 1.5 | 0.3 | 2/2 |

An initial inflammatory response is the essential initiator of the healing process, and leads to fibroblastic ingrowth. If the inflammatory response is excessive, pus is produced. Too little response, and no scar, or only temporary occlusion occurs. The Neuracryl M yielded a consistently greater inflammatory response than the present clinical standard, NBCA.

### Conclusion

Neuracryl M compares favorably with NBCA, showing greater penetration into the rete of the pig while being less likely to cause catheter trapping or distal embolization.

### EXAMPLE 17

### CLINICAL TRIAL COMPARING NEURACRYL M TO PVA FOAM

This example reports the results of a randomized clinical trial with 12 patients comparing Neuracryl M and PVA Foam.

### Materials and Methods

The procedure for the clinical study was conducted under the purview of the FDA and within approved institutional guidelines. Twelve pre-surgical patients satisfied the criteria to enter the study. After angiographic evaluation and randomization (75 percent to Neuracryl M/25 percent to PVA form according to the FDA recognized standard). Ten patients were treated using standard micro-catheter technique with Neuracyrl M; and two were treated with PVA. Patients were subsequently operated upon, and any residual nidus was removed. The angiograms were analyzed by three independent observers who compared the area of the nidus both before and after treatment in both AP and lateral planes.

### Results

No patient's condition worsened clinically. Of the two who randomized to PVA, one showed an 81 percent reduction in nidus size; the second had no significant change in nidus size. Of the ten who randomized to Neuracryl M, 5 were aniographically obliterated (using measurement criteria submitted to the FDA), 2 had nidus size reduction between 50 and 99 percent, 2 were obliterated less than 50 percent, and one nidus was judged to have actually enlarged by 25 percent. No catheter was glued in place.

### Conclusion

Neuracryl M provides a better angiographic obliteration rate than the historical standard, PVA form particles, and a better obliteration rate than the literature reports of patients treated with Histoaryl. In the patient whose AVM appeared to enlarge, the Neuracryl M slowed flow through the lesion sufficiently to make it more conspicuous and thus easier to measure.

### EXAMPLE 18

### INITIAL NEUROPATHOLOGIC OBSERVATION AFTER TREATMENT OF A HUMAN ARTERIOVENOUS MALFORMATION WITH NEURACRYL M

This example reports the treatment of a 34 year old male presented after acute hemorrhage of a right parieto-occipital arteriovenous malformation (AVM). The pathologic features of the AVM after embolization with Neuracryl M.

### Materials and Methods

Transcatheter embolization was performed on post-bleed day 21 using a novel, proprietary cyanoacrylate-based compound, Neuracryl M. Four days after embolization, the patient underwent surgical resection.

### Results

Scattered foci of Neuracryl M completely contained within vessels were observed. Embolic material formed a lamellar, spongiform configuration, was non-polarizable, and stained moderately ecsinophillic with hematoxylin and eosin. A profound acute inflammatory response was seen surround many of the AVM vessels, including frank necrosis and gome giant cell foreign body reaction.

### Conclusion

Neuracryl M was an effective embolic agent which was retained within vessel lumina and initiated a marked localized inflammatory reaction by 4 days. This report represents the initial pathologic observations of Neuracryl M in the first human subject treated. As further specimens are obtained, a more complete description of the behavior of Neuracryl M in the human brain will emerge.

## Claims

1. A composition comprising a combination of a monomer component comprised of an alkyl cyanoacrylate and at least one inhibitor; and a second component comprised of an alkyl cyanoacrylate monomer, an alkyl esterified fatty acid and an opacifying agent where said composition polymerizes when said composition contacts a non-ionic environment.

2. The composition of claim 1, wherein said alkyl cyanoacrylate is 2-hexyl cyanoacrylate.

3. The composition of claim 2, wherein said monomer component has at least two inhibitors.

4. The composition of claim 2, wherein said monomer component has at least three inhibitors.

5. The composition of claim 4, wherein one inhibitor is hydroquinone.

6. The composition of claim 5, wherein hydroquinone is in the range of about 50 to 150 PPM.

7. The composition of claim 4, wherein one inhibitor is *p*-methoxyphenol.

8. The composition of claim 7, wherein *p*-methoxyphenol is in the range of about 50 to 150 PPM.

9. The composition of claim 4, wherein one inhibitor is phosphoric acid.

10. The composition of claim 9, wherein phosphoric acid is in the range of about 125 to 375 PPM.

11. The composition of claim 8, wherein *p*-methoxyphenol is in the range of about 75 to 125 PPM.

12. The composition of claim 10, wherein phosphoric acid is in the range of about 187.5 to 312.5 . PPM.

13. The composition of claim 6 ,wherein hydroquinone is in the range of about 95 to 105 PPM.

14. The composition of claim 11, wherein p-methoxyphenol is in the range of about 95 to 105 PPM.

15. The composition of claim 12, wherein phosphoric acid is in the range of about 200 to 300 PPM.

16. The composition of anyone of claims 4-15, wherein said three inhibitors are hydroquinone, p-methoxyphenol and phosphoric acid.

17. The composition of anyone of claims 2-16, wherein said monomer is 2-hexyl cyanoacrylate.

18. The composition of claim 17, wherein said alkyl esterified fatty acid is selected from the group consisting of alkyl laurate, alkyl palmitate and stearic alkyl myristate.

19. The composition of claim 18, wherein said alkyl esterified fatty acid is ethyl myristate.

20. The composition of anyone of claims 17-19, wherein said opacifying agent is selected from the group consisting of gold, platinum, tantalum, titanium, tungsten and barium sulfate.

21. The composition of claim 20 wherein said opacifying agent is gold.

22. The composition of claim 20, wherein said gold is in fine powder form with individual particles no larger than about 7 µm in diameter.

23. The composition of claim 22 wherein said gold is in fine powder form with individual particles no larger than about 5 µm in diameter.

24. The composition of claim 23, wherein said gold is in fine powder form with individual particles no larger than about 2 µm in diameter.

25. The composition of claim 24, wherein said gold is in fine powder form with individual particles no larger than about 1 µm in diameter.

26. A composition comprising a combination of a monomer component comprised of 2-hexyl cyanoacrylate hydroquinone, *p*-methoxyphenol and phosphoric acid; and a second component comprised of 2-hexyl cyanoacrylate, ethyl myristate and gold, where said composition polymerizes when said composition contacts a non-ionic environment.

27. A composition comprising a combination of a monomer component comprised of an alkyl cyanoacrylate, at least one inhibitor; and a second component that functions as a opacificant agent and polymerization retardant where said composition polymerizes when said composition contacts a non-ionic environment.

28. The composition of claim 26, wherein said second component is a halogenated oil.

29. The composition of claim 26, wherein, said second component is Ethiodol^{®}.

30. A composition according to claim 27 comprising a monomer component comprised of 2-hexyl cyanoacrylate and hydroquinone, *p*-methoxyphenol and phosphoric acid, and Ethiodol^{®}.

31. Use of the composition according to claims 1 or 27 for preparing a pharmaceutical product for filling, occluding, partially filling or partially occluding an unfilled volume or space in a mass in a non-ionic environment.

## Patentansprüche

1. Zusammensetzung, umfassend eine Kombination einer Monomer-Komponente, die ein Alkylcyanoacrylat und mindestens einen Inhibitor umfasst; und eine zweite Komponente, die ein Alkylcyanoacrylat-Monomer, einen Fettsäure-alkylester und ein strahlungsundurchlässiges Agens umfasst, wobei die Zusammensetzung polymerisiert, wenn die Zusammensetzung mit einer nicht-ionischen Umgebung in Kontakt kommt.

2. Zusammensetzung nach Anspruch 1, wobei das Alkylcyanoacrylat 2-Hexylcyanoacrylat ist.

3. Zusammensetzung nach Anspruch 2, wobei die Monomer-Komponente mindestens zwei Inhibitoren hat.

4. Zusammensetzung nach Anspruch 2, wobei die Monomer-Komponente mindestens drei Inhibitoren hat.

5. Zusammensetzung nach Anspruch 4, wobei ein Inhibitor Hydrochinon ist.

6. Zusammensetzung nach Anspruch 5, wobei das Hydrochinon im Bereich von etwa 50 bis 150 ppm liegt.

7. Verbindung nach Anspruch 4, wobei ein Inhibitor *p*-Methoxyphenol ist.

8. Zusammensetzung nach Anspruch 7, wobei *p*-Methoxyphenol im Bereich von etwa 50 bis 150 ppm liegt.

9. Zusammensetzung nach Anspruch 4, wobei ein Inhibitor Phosphorsäure ist.

10. Zusammensetzung nach Anspruch 9, wobei Phosphorsäure im Bereich von etwa 125 bis 375 ppm liegt.

11. Zusammensetzung nach Anspruch 8, wobei *p*-Methoxyphenol im Bereich von etwa 75 bis 125 ppm liegt.

12. Zusammensetzung nach Anspruch 10, wobei Phosphorsäure im Bereich von etwa 187,5 bis 312,5 ppm liegt.

13. Zusammensetzung nach Anspruch 6, wobei Hydrochinon im Bereich von etwa 95 bis 105 ppm liegt.

14. Zusammensetzung nach Anspruch 11, wobei *p*-Methoxyphenol im Bereich von etwa 95 bis 105 ppm liegt.

15. Zusammensetzung nach Anspruch 12, wobei Phosphorsäure im Bereich von etwa 200 bis 300 ppm liegt.

16. Zusammensetzung nach einem der Ansprüche 4 bis 15, wobei die drei Inhibitoren Hydrochinon, *p*-Methoxyphenol und Phosphorsäure sind.

17. Zusammensetzung nach einem der Ansprüche 2 bis 16, wobei das Monomer 2-Hexylcyanoacrylat ist.

18. Zusammensetzung nach Anspruch 17, wobei der Fettsäurealkylester ausgewählt ist aus der Gruppe bestehend aus Alkyllaurat, Alkylpalmitat und Stearin-Alkylmyristat.

19. Zusammensetzung nach Anspruch 18, wobei der Fettsäurealkylester Ethylmyristat ist.

20. Zusammensetzung nach einem der Ansprüche 17 bis 19, wobei das strahlungsundurchlässige Agens ausgewählt ist aus der Gruppe bestehend aus Gold, Platin, Tantal, Titan, Wolfram und Bariumsulfat.

21. Zusammensetzung nach Anspruch 20, wobei das strahlungsundurchlässige Agens Gold ist.

22. Zusammensetzung nach Anspruch 20, wobei das Gold in feiner Pulverform vorliegt, mit einzelnen Partikeln, die nicht größer als etwa 7 µm im Durchmesser sind.

23. Zusammensetzung nach Anspruch 22, wobei das Gold in feiner Pulverform vorliegt, mit einzelnen Partikeln, die nicht größer als ungefähr 5 µm im Durchmesser sind.

24. Zusammensetzung nach Anspruch 23, wobei das Gold in feiner Pulverform vorliegt, mit einzelnen Partikeln, die nicht größer als ungefähr 2 µm im Durchmesser sind.

25. Zusammensetzung nach Anspruch 24, wobei das Gold in feiner Pulverform vorliegt, mit einzelnen Partikeln, die nicht größer als ungefähr 1 µm im Durchmesser sind.

26. Zusammensetzung, umfassend eine Kombination aus einer Monomer-Komponente, die 2-Hexylcyanoacrylat, Hydrochinon, *p*-Methoxyphenol und Phosphorsäure umfasst, und einer zweiten Komponente, die 2-Hexylcyanoacrylat, Ethylmyristat und Gold umfasst, wobei die Zusammensetzung polymerisiert, wenn die Zusammensetzung mit einer nicht-ionischen Umgebung in Kontakt kommt.

27. Zusammensetzung, umfassend eine Kombination aus einer Monomer-Komponente, die ein Alkylcyanoacrylat und mindestens einen Inhibitor umfasst; und einer zweiten Komponente, die als strahlungsundurchlässiges Agens und Polymerisationshemmer funktioniert, wobei die Zusammensetzung polymerisiert, wenn die Zusammensetzung mit einer nicht-ionischen Umgebung in Kontakt kommt.

28. Zusammensetzung nach Anspruch 26, wobei die zweite Komponente ein halogeniertes Öl ist.

29. Zusammensetzung nach Anspruch 26, wobei die zweite Komponente Ethiodol® ist.

30. Zusammensetzung nach Anspruch 27, umfassend eine Monomer-Komponente, die 2-Hexylcyanoacrylat und Hydrochinon, *p*-Methoxyphenol und Phosphorsäure umfasst, und Ethiodol^{®}.

31. Verwendung der Zusammensetzung nach einem der Ansprüche 1 bis 27 für die Herstellung eines pharmazeutischen Produktes zum Füllen, Verstopfen, partiellen Füllen oder partiellen Verstopfen eines nicht-gefüllten Volumens oder Raumes in einer Masse in einer nicht-ionischen Umgebung.

## Revendications

1. Composition comprenant une combinaison d'un composant monomère composé d'un cyanoacrylate d'alkyle et d'au moins un inhibiteur ; et d'un second composant composé d'un monomère de cyanoacrylate d'alkyle, d'un ester alkyle d'acide gras et d'un agent opacifiant où ladite composition polymérise lorsque ladite composition entre en contact avec un environnement non-ionique.

2. Composition selon la revendication 1, dans laquelle ledit cyanoacrylate d'alkyle est le cyanoacrylate de 2-hexyle.

3. Composition selon la revendication 2, dans laquelle ledit composant monomère a au moins deux inhibiteurs.

4. Composition selon la revendication 2, dans laquelle ledit composant monomère a au moins trois inhibiteurs.

5. Composition selon la revendication 4, dans laquelle un inhibiteur est l'hydroquinone.

6. Composition selon la revendication 5, dans laquelle l'hydroquinone est dans l'intervalle d'environ 50 à 150 PPM.

7. Composition selon la revendication 4, dans laquelle un inhibiteur est le p-méthoxyphénol.

8. Composition selon la revendication 7, dans laquelle le p-méthoxyphénol est dans l'intervalle d'environ 50 à 150 PPM.

9. Composition selon la revendication 4, dans laquelle un inhibiteur est l'acide phosphorique.

10. Composition selon la revendication 9, dans laquelle l'acide phosphorique est dans l'intervalle d'environ 125 à 375 PPM.

11. Composition selon la revendication 8, dans laquelle le p-méthoxyphénol est dans l'intervalle d'environ 75 à 125 PPM.

12. Composition selon la revendication 10, dans laquelle l'acide phosphorique est dans l'intervalle d'environ 187,5 à 312,5 PPM.

13. Composition selon la revendication 6, dans laquelle l'hydroquinone est dans l'intervalle d'environ 95 à 105 PPM.

14. Composition selon la revendication 11, dans laquelle le p-méthoxyphénol est dans l'intervalle d'environ 95 à 105 PPM.

15. Composition selon la revendication 12, dans laquelle l'acide phosphorique est dans l'intervalle d'environ 200 à 300 PPM.

16. Composition selon l'une quelconque des revendications 4-15, dans laquelle lesdits trois inhibiteurs sont l'hydroquinone, le p-méthoxyphénol et l'acide phosphorique.

17. Composition selon l'une quelconque des revendications 2-16, dans laquelle ledit monomère est le cyanoacrylate de 2-hexyle.

18. Composition selon la revendication 17, dans laquelle ledit ester alkyle d'acide gras est choisi dans le groupe constitué de laurate d'alkyle, palmitate d'alkyle et myristate d'alkyle stéarique.

19. Composition selon la revendication 18, dans laquelle ledit ester alkyle d'acide gras est le myristate d'éthyle.

20. Composition selon l'une quelconque des revendications 17-19, dans laquelle ledit agent opacifiant est choisi dans le groupe constitué de l'or, platine, tantale, titane, tungstène et sulfate de baryum.

21. Composition selon la revendication 20, dans lequel ledit agent opacifiant est l'or.

22. Composition selon la revendication 20, dans laquelle ledit or est sous la forme d'une poudre fine avec des particules individuelles de diamètre inférieur ou égal à environ 7 µm.

23. Composition selon la revendication 22, dans laquelle ledit or est sous la forme d'une poudre fine avec des particules individuelles de diamètre inférieur ou égal à environ 5 µm.

24. Composition selon la revendication 23, dans laquelle ledit or est sous la forme d'une poudre fine avec des particules individuelles de diamètre inférieur ou égal à environ 2 µm.

25. Composition selon la revendication 24, dans laquelle ledit or est sous la forme d'une poudre fine avec des particules individuelles de diamètre inférieur ou égal à environ 1 µm.

26. Composition comprenant une combinaison d'un composant monomère composé de cyanoacrylate de 2-hexyle, d'hydroquinone, de p-méthoxyphénol et d'acide phosphorique ; et d'un second composant composé de cyanoacrylate de 2-héxyle, de myristate d'éthyle et d'or, où ladite composition polimérise lorsque ladite composition entre en contacte avec un environnement non-ionique.

27. Composition comprenant une combinaison d'un composant monomère composé d'un cyanoacrylate d'alkyle, d'au moins un inhibiteur ; et un second composant qui fonctionne comme un agent opacifiant et agent retardant, où ladite composition polymérise lorsque ladite composition entre en contact avec un environnement non-ionique.

28. Composition selon la revendication 26, dans laquelle ledit second composant est une huile halogénée.

29. Composition selon la revendication 26, dans laquelle ledit second composant est l'Ethiodol ®.

30. Composition selon la revendication 27, comprenant un composant monomère composé de cyanoacrylate de 2-hexyle et d'hydroquinone, de p-méthoxyphénol et d'acide phosphorique, et d'Ethiodol ®.

31. Utilisation de la composition selon la revendication 1 ou 27 pour préparer un produit pharmaceutique pour remplir, boucher, remplir partiellement ou boucher partiellement un volume ou espace non-rempli dans une masse dans un environnement non-ionique.
